(19) Europäisches Patentamt
European Patent Office
Office européen des brevets

(11) Veröffentlichungsnummer : **0 506 704 B1**

# (12) EUROPÄISCHE PATENTSCHRIFT

(45) Veröffentlichungstag der Patentschrift :
**23.03.94 Patentblatt 94/12**

(51) Int. Cl.$^5$ : **A61K 31/685,** C07F 9/09, C07F 9/10, C07F 9/165

(21) Anmeldenummer : **91900254.3**

(22) Anmeldetag : **05.12.90**

(86) Internationale Anmeldenummer :
**PCT/EP90/02100**

(87) Internationale Veröffentlichungsnummer :
**WO 91/09602 11.07.91 Gazette 91/15**

## (54) VERWENDUNG VON ALKYLPHOSPHOLIPIDEN ALS ANTIVIRALE ARZNEIMITTEL, SOWIE NEUE VERBINDUNGEN.

(30) Priorität : **23.12.89 DE 3942933**

(43) Veröffentlichungstag der Anmeldung :
**07.10.92 Patentblatt 92/41**

(45) Bekanntmachung des Hinweises auf die Patenterteilung :
**23.03.94 Patentblatt 94/12**

(84) Benannte Vertragsstaaten :
**AT CH DE FR GB IT LI**

(56) Entgegenhaltungen :
**EP-A- 0 108 565**
**WO-A-87/01592**
**Pharmazie, Band 41, Nr. 6, 1986, D. Sandow et al; Seiten 404-406**
**Microbios, Band 51, 1987, The Faculty Press, Cambridge, GB, G. Menzel et al.:"Aktivity of alkyl phospholipids in virus-host systems" p. 15-21**
**Patent abstr. of JP, Bd 11, Nr. 353**

(73) Patentinhaber : **BOEHRINGER MANNHEIM GMBH**
**D-68298 Mannheim (DE)**

(72) Erfinder : **ZILCH, Harald**
**Alsenweg 24**
**D-6800 Mannheim 31 (DE)**
Erfinder : **BOSIES, Elmar**
**Delpstrasse 11**
**D-6940 Weinheim (DE)**
Erfinder : **HERRMANN, Dieter**
**An der Neckarspitze 13**
**D-6900 Heidelberg (DE)**
Erfinder : **KOCH, Edith**
**Langonerstrasse 18**
**D-8122 Penzberg (DE)**

(74) Vertreter : **Weber, Manfred, Dr. et al**
**c/o Boehringer Mannheim GmbH,**
**Patentabteilung, Sandhoferstrasse 116**
**D-68298 Mannheim (DE)**

**EP 0 506 704 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Neue Alkylphospholipide und antivirale Arzneimittel

enthaltend diese Verbindungen Gegenstand der vorliegenden Erfindung sind neue Alkylphospholipide und antivirale Arzneimittel enthaltend diese Verbindungen.

Die vorliegende Erfindung betrifft neue Verbindungen mit antiviraler Wirkung. Es hat sich gezeigt, daß insbesondere Phospholipide der Formel I

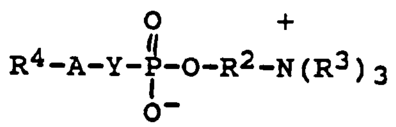 (I)

eine gute antivirale Wirkung aufweisen, in der

$R^4$ einen geradkettigen oder verzweigten, gesättigten oder ungesättigten aliphatischen Rest mit 7-28 Kohlenstoffatomen bedeutet,

A die Gruppe

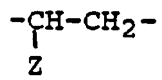 , 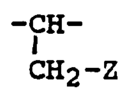 oder 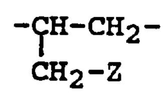

bedeutet, und

Z eine $C_1$-$C_{20}$-Alkoxycarbonyl-, $C_1$-$C_{20}$-Alkylcarbonyl-, $C_1$-$C_{20}$ Alkylaminocarbonyl-, $C_1$-$C_{20}$-Alkylmercapto-, $C_1$-$C_{20}$-Alkansulfinyl-, $C_1$-$C_{20}$-Alkansulfonyl- oder $C_8$-$C_{15}$-Alkoxygruppe darstellt, wobei für den Fall, daß $R^4$ eine geradkettige Alkylgruppe mit 7-18 C-Atomen bedeutet, Z auch eine unverzweigte Alkylgruppe mit 9-13 C-Atomen sein kann,

$R^2$ eine geradkettige oder verzweigte Alkylenkette mit 2-4 Kohlenstoffatomen,

$R^3$ Wasserstoff oder eine $C_1$-$C_6$-Alkylgruppe und

Y ein Sauerstoff- oder Schwefelatom

bedeuten, wobei die Verbindungen bezüglich der Definition $R^4$-A-nicht mehr als maximal 33 Kohlenstoffatome besitzen, sowie deren pharmakologisch unbedenkliche Salze und optische Isomere, mit Ausnahme der Verbindungen 2-Decyl-Tetradecyl-1-phosphorsäuremonocholinester und 2-Tetradecyl-octadecano-1-phosphocholin.

In der Deutschen Offenlegungsschrift DE-A-3,304,870 werden Alkyl-phospholipide mit ähnlicher Struktur beschrieben, sowie deren Verwendung zur Herstellung von Arzneimitteln. Es wird eine cancerostatische Wirkung dieser Phospholipide angegeben. Diese Derivate eignen sich insbesondere zur Herstellung von Antitumormitteln.

In der EP-A-300,397 sind Hydroxy-, $C_1$-$C_6$-Alkoxy- und Benzyloxysubstituierte Phospholipide als Phospholipidase-A2-Inhibitoren beschrieben.

In der JP 62/126 192 wird die Herstellung von strukturell ähnlichen Alkylphospholipid-Derivaten beschrieben. Insbesondere ist aus dieser Anmeldung die Verbindung 2-Decyl-tetradecyl-l-phosphorsäuremonocholinester bereits vorbekannt.

Aus dem Artikel von Lee et al. in "Neurochemistry International, Band 4, Nr. 5, S. 355-359 (1982) ist die Verbindung 2-Tetradecyloctadecano-1-phosphocholin bekannt.

Aus der JP 61/24 530 sind kurzkettige Alkoxy[[[(trialkylammonio)ethoxy]phosphinyl]oxy]alkanhydroxide in Form ihrer inneren Salze mit antitumoraler, antiinflammatorischer und antihypertensiver Wirkung bekannt.

Die EP-A-108,565 beschreibt Alkylphospholipide mit antitumoraler, fungizider und antiparasitärer Wirkung.

Es wurde nun überraschenderweise gefunden, daß die neuen Alkylphospholipide der Formel I eine ausgeprägte antivirale Wirkung aufweisen, und sich daher besonders gut zur Behandlung von viralen bzw. retroviralen Infektionen eignen. Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Aquired Immune Deficiency Syndrom (AIDS), den AIDS-related-complex (ARC) und deren Vorstadien, Herpes-,

Cytomegalie-Virus (CMV)-, Influenzaund andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxy-thymidin (AZT), bekannt als Zidovudine oder Retrovir$^R$, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J.Infec.Dis. 157, 427-431).

Es besteht daher ein sehr großes Bedürfnis an Chemotherapeutika, die möglichst spezifisch mit viral oder retroviral bedingten Erkrankungen oder deren Symptomen interferieren, ohne jedoch die anderen, normal ablaufenden natürlichen Körperfunktionen zu beeinflussen.

Der vorliegenden Erfindung lag daher die Aufgabe zugrunde, verbesserte antiviral wirksame Arzneimittel zur Verfügung zu stellen.

Die Alkylphospholipide der Formel I hemmen sehr spezifisch die Virusvermehrung, wobei insbesondere folgende Viren zu nennen sind: HIV, Herpes, Sendai, Cytomegalie (CMV), Influenza, Parainfluenza, Epstein-Barr (EBV), Vesikular Stomatitis Virus (VSV), Hepatitis, Meningitis, Enzephalitis, etc.. Die genannten Verbindungen können vorteilhaft prophylaktisch oder therapeutisch bei der Behandlung von allen Krankheiten eingesetzt werden, bei denen eine virale bzw. retrovirale Infektion von pathophysiologischer, symptomatischer oder klinischer Relevanz ist.

In der allgemeinen Formel I zeigen insbesondere solche Phospholipide eine gute antivirale Wirkung, wenn sie durch die folgenden Merkmale gekennzeichnet sind:

$R^4$ ist vorzugsweise eine geradkettige $C_7$-$C_{28}$-Alkyl-, insbesondere $C_8$-$C_{15}$- oder $C_9$-$C_{14}$-Alkylgruppe, wie beispielsweise die Octyl-, Decyl-, Dodecyl-, Tridecyl-, Tetradecylgruppe, wobei die Decyl-, Undecyl-, Dodecyl- und Tridecylgruppe besonders bevorzugt sind.

Z ist vorzugsweise $C_1$-$C_{20}$-Alkylmercapto-, $C_1$-$C_{20}$-Alkansulfinyl-, $C_1$-$C_{20}$-Alkansulfonyl-, $C_8$-$C_{15}$-Alkoxy-, $C_1$-$C_{20}$-Alkoxycarbonyl-, $C_1$-$C_{20}$-Alkylcarbonyl- oder $C_1$-$C_{20}$-Alkylaminocarbonylgruppe in Frage, oder für den Fall, daß $R^4$ eine geradkettige Alkylgruppe mit 7-18 C-Atomen bedeutet, kann Z auch eine unverzweigte $C_9$-$C_{13}$-Alkylgruppe sein. Bevorzugt enthalten die Alkyl"-teile der zuvor genannten Gruppen mindestens 8, insbesondere mindestens 10 C-Atome und höchstens bis zu 15, insbesondere bis zu 13 C-Atome, wie z.B. die Octyl-, Nonyl-, Decyl-, Undecyl- oder Dodecylkomponente. $R^4$ ist bevorzugt eine geradkettige Alkylgruppe mit 7-14 Kohlenstoffatomen. $R^2$ bedeutet insbesondere eine geradkettige oder verzweigte $C_2$-$C_4$-Alkylengruppe, wobei die Ethylengruppe bevorzugt ist.

Für $R^3$ kommt insbesondere ein Wasserstoffatom oder eine $C_1$-$C_4$-Alkylgruppe, vorzugsweise die Methylgruppe in Frage.

Unter der Definition der $-N^+(R^3)_3$-Gruppe in der Formel I sollen sowohl die identisch substituierten Stickstoffderivate, wie z.B. die Trimethylammoniumgruppe, als auch die gemischt substituierten Derivate, wie z.B. Dimethylammonium-, Diethylammonium-, n-Butyl-dimethylammonium- oder Methyl-di-ethylammoniumgruppe verstanden werden bzw. alle durch die Kombination der in der Definition von $R^3$ genannten Gruppen möglichen Varianten.

Besonders bevorzugte Verbindungen der allgemeinen Formel I sind solche, in denen die Gruppe $R^4$ eine $C_7$-$C_{14}$-Alkylgruppe darstellt, wie beispielsweise die Octyl-, Nonyl-, Decyl-, Undecyl-, Dodecyl-, Tridecyl- oder Tetradecylgruppe und Z eine $C_8$-$C_{15}$-Alkylmercapto-, $C_8$-$C_{15}$-Alkoxy-, $C_8$-$C_{15}$-Alkoxycarbonyl-, $C_8$-$C_{15}$-Alkylcarbonyl-, $C_8$-$C_{15}$-Alkylaminocarbonyl- oder $C_8$-$C_{15}$-Alkylcarbonylaminogruppe, wie beispielsweise die Decylmercapto-, Undecylmercapto-, Decyloxy-, Undecyloxy-, Dodecyloxy-, Decyloxycarbonyl-, Undecylcarbonyl-, Decylaminocarbonyl- oder Decylcarbonylaminogruppe.

Zur Herstellung der Verbindungen der Formel I eignet sich vorzugsweise das folgende Verfahren:

Eine Verbindung der allgemeinen Formel II

$$R^4\text{-A-YH} \qquad \text{(II)}$$

in der

$R^4$, A und Y die oben angegebene Bedeutung haben,

wird mit einer Verbindung der allgemeinen Formel III

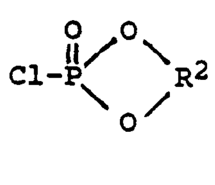

(III)

in der

$R^2$ die oben genannte Bedeutung hat, in Gegenwart eines säurebindenden Mittels umsetzt und das Reaktionsprodukt direkt mit gegebenenfalls alkyliertem Ammoniak behandelt.

Das Verfahren wird in der Regel so durchgeführt, daß ein Alkanol bzw. Alkanthiol der allgemeinen Formel II mit einer Verbindung der allgemeinen Formel III in Gegenwart eines säurebindenden Mittels, wie z.B. Triethylamin oder Pyridin in einem absoluten, inerten organischen Lösungsmittel, wie z.B. chlorierter Kohlenwasserstoff oder Toluol, bei Temperaturen um den Gefrierpunkt bis Raumtemperatur reagiert, und das Reaktionsprodukt direkt mit gegebenenfalls alkyliertem Ammoniak behandelt wird. Dazu löst man den Ammoniak oder das Alkylamin in einem Medium, das sowohl den Phosphorsäure-diester als auch Ammoniak oder das Amin genügend gut löst, wozu sich besonders Mischungen aus Acetonitril oder niederen Alkoholen mit chlorierten Kohlenwasserstoffen eignen und vervollständigt die Reaktion bei einer Temperatur von 20 bis 70°C.

Man kann auch stufenweise vorgehen, also zuerst eine Alkylammoniumgruppe einführen und mit Alkylhalogenid anschließend zum Di- oder Trialkylammoniumalkylester umsetzen.

Sämtliche Zwischenstufen sowie Endprodukte lassen sich bequem säulenchromatographisch mit üblichen Elutionsmitteln, wie z.B. Ether, Ligroin, chlorierten Kohlenwasserstoffen, niederen Alkoholen oder Mischungen derselben, an Kieselgel reinigen, wobei im Falle des betainartigen Endprodukts zweckmässig etwas Wasser zugesetzt wird.

Die pharmakologisch verträglichen Salze erhält man in üblicher Weise, z.B. durch Neutralisation der Verbindungen der Formel I mit nichttoxischen anorganischen oder organischen Säuren, wie z.B. Salz-, Schwefel-, Phosphor-, Bromwasserstoff-, Essig-, Milch-, Zitronen-, Äpfel-, Salicyl-, Malon-, Malein- oder Bernsteinsäure.

Die Arzneimittel enthaltend Verbindungen der Formel I zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragées, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylendiamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage:

1. 2-Undecylmercapto-tetradecyl-1-phosphorsäuremonocholinester
2. 2-Decyloxy-pentadecyl-1-phosphorsäuremonocholinester
3. 2-Undecylmercapto-pentadecyl-1-phosphorsäuremonocholinester
4. 2-Undecyloxy-hexadecyl-1-phosphorsäuremonocholinester
5. 2-Dodecylsulfinyl-tetradecyl-1-phosphorsäuremonocholinester
6. 2-Undecylmercapto-tridecyl-1-phosphorsäuremonocholinester
7. 2-Undecyloxymethyl-undecyl-1-phosphorsäuremonocholinester
8. 2-Undecylmercaptomethyl-dodecyl-1-phosphorsäuremonocholinester
9. 2-Undecylsulfinylmethyl-tridecyl-1-phosphorsäuremonocholinester
10. 2-Dodecylmercaptomethyl-dodecyl-1-phosphorsäuremonocholinester
11. 2-Undecyloxymethyl-tetradecyl-1-phosphorsäuremonocholinester
12. 2-Decylsulfonylmethyl-tridecyl-1-phosphorsäuremonocholinester
13. 2-Decyloxymethyl-dodecyl-1-phosphorsäuremonocholinester
14. 1-Undecyloxy-tridecyl-2-phosphorsäuremonocholinester
15. 1-Undecyloxy-tetradecyl-2-phosphorsäuremonocholinester
16. 1-Decylmercapto-pentadecyl-2-phosphorsäuremonocholinester
17. 1-Dodecylmercapto-tridecyl-2-phosphorsäuremonocholinester
18. 1-Undecylmercapto-tetradecyl-2-phosphorsäuremonocholinester

19. 1-Undecylmercapto-tridecyl-2-phosphorsäuremonocholinester
20. 1-Decylsulfinyl-tetradecyl-2-phosphorsäuremonocholinester
21. 1-Undecylsulfonyl-tridecyl-2-phosphorsäuremanocholinester
22. 2-Decyl-dodecyl-1-phosphorsäuremonocholinester
23. 2-Undecyl-tridecyl-1-phosphorsäuremonocholinester
24. 2-Dodecyl-dodecyl-1-phosphorsäuremonocholinester
25. 2-Decyl-tetradecyl-1-phosphorsäuremonocholinester
26. 2-Undecyl-dodecyl-1-phosphorsäuremonocholinester
27.2-Decyl-tridecyl-1-phosphorsäuremonocholinester
28. 2-Decyl-hexadecyl-1-phosphorsäuremonocholinester
29. 2-Decyl-pentadecyl-1-phosphorsäuremonocholinester
30. Eicosyl-10-phosphorsäuremonocholinester
31. Heneicosyl-11-phosphorsäuremonocholinester
32. Docosyl-11-phosphorsäuremonocholinester
33. Tricosyl-12-phosphorsäuremonocholinester
34. Tetracosyl-12-phosphorsäuremonocholinester
35. Pentacosyl-13-phosphorsäuremonocholinester
36. Tricosyl-11-phosphorsäuremonocholinester
37. 2-(2-Oxodecan-1-yl)-tridecyl-1-phosphorsäuremonocholinester
38. 2-(2-Oxotridecan-1-yl)tetradecyl-1-phosphorsäuremonocholinester
39. 2-(2-Oxododecan-1-yl)pentadecyl-1-phosphorsäuremonocholinester

Beispiel 1

2-Dodecylmercapto-tetradecyl-1-phosphorsäure-monocholinester

a) 2-Dodecylmercapto-tetradecansäuremethylester

Eine Natriummethylatlösung aus 1.2 g (52 mmol) Natrium in 50 ml Methanol wurde mit 12.5 ml (52 mmol) 1-Dodecylmercaptan in 30 ml Methanol versetzt und 1 h bei Raumtemperatur gerührt. Dann wurden 15 g (47 mmol) 2-Bromtetradecansäuremethylester in 40 ml Methanol zugetropft und die sich bildende Suspension weitere 24 h bei Raumtemperatur gerührt. Nach dieser Zeit wurde der Niederschlag abgesaugt, das Lösungsmittel vom Filtrat entfernt und der Rückstand nach Aufnehmen in Dichlormethan mit Wasser gewaschen. Nach Eindampfen der organischen Phase erhielt man ein nach DC (Ether/Hexan 0.3/10) schwach verunreinigtes Rohprodukt in fast quantitativer Ausbeute, das ohne weitere Reinigung in die nächste Reaktion eingesetzt wurde.

b) 2-Dodecylmercapto-tetradecanol-1

Das Rohprodukt der letzten Reaktion wurde in 200 ml abs. Ether gelöst und so zu einer Suspension aus 1.25 g (33 mmol) Lithiumaluminiumhydrid in 190 ml abs. Ether getropft, daß der Ether gelinde siedete. Nach vollständiger Zugabe wurde eine weitere Stunde unter Rückfluß erhitzt, anschließend im Eisbad abgekühlt und nacheinander langsam mit 1.5 ml Wasser, 1.2 ml 5 N NaOH und nochmals 5.7 ml Wasser versetzt. Der Niederschlag konnte 30 min später abgesaugt werden, das Filtrat wurde eingedampft und der Rückstand anschließend durch Säulenchromatographie an Kieselgel 60 mit Ether/Isohexan 1/10 als Eluens gereinigt. Man erhielt 16.9 g (87 %, bezogen auf eingesetzten 2-Bromtetradecansäuremethylester) als farbloses Öl.

c) 2-Dodecylmercaptotetradecyl-1-phosphorsäure-monocholinester

8 g (19,3 mmol) 2-Dodecylmercapto-tetradecanol-1 in 55 ml Dichlormethan sowie 7,5 ml Triethylamin wurden bei -25°C mit einer Lösung aus 3.6 g (25 mmol) 2-Chlor-2-oxo-1,3,2-dioxaphospholan (Fluka) in 15 ml Dichlormethan versetzt und 30 Minuten gerührt. Dann ließ man auf RT erwärmen, entfernte das Lösungsmittel im Rotationsverdampfer und suspendierte den Rückstand in Ether. Nach Abtrennen das Triethylammoniumsalzes wurde das Filtrat erneut eingedampft und der ölige Rückstand in 45 ml Acetonitril aufgenommen. Die klare Lösung wurde durch Einleiten bei 20°C mit Trimethylamin gesättigt und 96 Stunden bei RT im geschlossenen Gefäß gerührt. Danach wurde der Niederschlag abgesaugt, mit Acetonitril gewaschen und aus Dichlormethan/Aceton umgefällt. Ausb. 5.37 g (48 % d.Th.), Schmp. 208-213°C.

Beispiel 2

2-Dodecyloxytetradecvl-1-phosphorsäure-monocholinester wurde analog zu Bsp. 1 hergestellt und durch Säulenchromatographie an Kieselgel 60 mit Dichlormethan/Methanol/Wasser 6,5/2,5/0,4 als Eluens gereinigt. Ausbeute 34%, Schmp. 223-225°C (Zers.). Das als Edukt verwendete 2-Dodecyloxy-tetradecanol-1 wurde aus

2-Bromtetradecansäuremethylester durch Umsetzung mit Natrium-dodecanolat und Reduktion des Rohproduktes mit Lithiumaluminiumhydrid in Ether hergestellt. Ausbeute 48%, bezogen auf den eingesetzten 2-Bromtetradecansäuremethylester.

Beispiel 3

2-Dodecylmercaptomethyl-tridecyl-1-phosphorsäure-monocholinester

a) Undecylmalonsäurediethylester

Zu einer Suspension aus 2.65 g (105 mmol) Natriumhydrid 95% in 60 ml abs. DMF wurden langsam 16 g (100 mmol) Malonsäurediethylester in 70 ml abs. DMF getropft und 30 min. nachgerührt. Dann wurden 24 ml (105 mmol) Undecylbromid in 150 ml Toluol zugegeben und die sich bildende Suspension 24 h bei Raumtemperatur gerührt. Das Lösungsmittel wurde im Vakuum entfernt, der Rückstand mit Wasser versetzt und mehrfach mit Ether extrahiert. Die vereinigten Etherextrakte wurden getrocknet, eingedampft und der Rückstand ohne weitere Reinigung in die nächste Reaktion eingesetzt.

b) 2-Hydroxymethyl-tridecanol-1

Das Rohprodukt der letzten Reaktion in 240 ml abs. Ether wurde so zu einer Suspension aus 5,6 g (148 mmol) Lithiumaluminiumhydrid in 440 ml abs. Ether getropft, daß der Ether gelinde siedete. Nach vollständiger Zugabe wurde weitere 30 min. unter Rückfluß erhitzt, anschließend im Eisbad abgekühlt und nacheinander langsam mit 6 ml Wasser, 4,3 ml 5n NaOH und nochmals 20.4 ml Wasser versetzt. Nach 30 min. wurde der Niederschlag abgesaugt, mit Ether gewaschen und das Filtrat vom Lösungsmittel befreit. Der Rückstand wurde durch Flash-Säulenchromatographie mit Ether/Isohexan 2/1 als Eluens gereinigt. Ausbeute 13.6 g (59%, bezogen auf Malonsäurediethylester), Schmp. 62-65°C.

c) 2-Hydroxymethyl-tridecanol-1-benzolsulfonat

9.5 g (41 mmol) des Diols der letzten Reaktion wurden in 50 ml abs. Pyridin gelöst, bei o°C langsam mit 5.55 ml (43,5 mmol) Benzolsulfonsäurechlorid versetzt und 1 h im Eisbad nachgerührt. Danach wurde der Ansatz auf Eis gegossen, mit 6n HCl angesäuert und mit Dichlormethan extrahiert. Nach Trocknung über $Na_2SO_4$ wurde die organische Phase eingedampft und der Rückstand durch Säulenchromatographie an Kieselgel 60 mit Ether/Isohexan 1/1 als Eluens gereinigt. Ausbeute 8.2 g (54%) als öl.

d) 2-Dodecylmercaptomethyl-tridecanol-1

5.8 ml (24.3 mmol) Dodecylmercaptan wurden zu einer Lösung aus 1.35 KOH in 60 ml Ethanol getropft, lh bei Raumtemperatur gerührt und mit 8.1 g (22 mmol) des Benzolsulfonats der letzten Reaktion in 27 ml Ethanol versetzt. Nach 16 h Rühren bei Raumtemperatur wurde die Suspension auf Eis gegossen, mit Salzsäure angesäuert und mehrfach mit Dichlormethan extrahiert. Die vereinigten organischen Phasen wurden über $Na_2SO_4$ getrocknet und von Lösungsmitteln befreit. Ausbeute fast quantitativ (Öl).

e) 2-Dodecylmercaptomethyl-tridecyl-1-phosphorsäure-monocholinester

Die Verbindung wurde analog zu Bsp. 1 und 2 aus dem 2-Dodecylmercaptomethyl-tridecanol-1 hergestellt. Ausbeute 82%, Schmp. 226-228°C (Zers.).

Beispiel 4

2 -Decyloxy-tetradecyl-1-phosphorsäure-monocholinester wurde analog zu den Beispielen 1 und 2 hergestellt und durch Säulenchromatographie an Kieselgel 60 mit Dichlormethan/Methanol/Wasser 6.5/2.5/0.4 als Eluens gereinigt. Ausbeute 43 %, Schmp. 184-194 oC (langsame Zersetzung).

Das als Edukt eingesetzte 2-Decyloxy-tetradecanol-l wurde analog zu Beispiel 2 in 53 % Ausbeute hergestellt.

Beispiel 5

2-Dodecyloxy-pentadecyl-1-phosphorsäure-monocholinester wurde analog den Beispielen 1, 2 und 4 hergestellt und durch Säulenchromatographie an Kieselgel 60 mit Dichlormethan/Methanol/Wasser 6.5/2.5/0.4 als Eluens gereinigt. Ausbeute 31 %, Schmp. >230 oC (Zersetzung).

Das als Edukt eingesetzte 2-Dodecyloxy-pentadecanol-l wurde analog zu Beispiel 2 in 42 % Ausbeute hergestellt.

Beispiel 6

2-Dodecyloxy-tridecyl-1-phosphorsäure-monocholinester wurde analog den Beispielen 1, 2, 4 und 5 hergestellt und durch Säulenchromatograpie an Kieselgel 60 mit Dichlormethan/Methanol/Wasser 6.5/2.5/0.4 als

Eluens gereinigt. Ausbeute 49 %, Schmp. 218-223 oC (Zersetzung).

Das als Eluens eingesetzte 2-Dodecyloxy-tridecanol-l wurde anaolg zu Beispiel 2 in 61 % Ausbeute hergestellt.

Beispiel 7

Untersuchungen zur pharmakologischen Wirksamkeit

Die Verbindungen der vorliegenden Erfindung weisen eine antivirale Wirksamkeit auf und können somit beispielsweise zur Behandlung von HIV-bedingten Krankheiten oder Infektionen eingesetzt werden. Der Nachweis der Anti-HIV-Wirksamkeit erfolgt in Zell- oder Gewebekulturen, wobei die direkte Hemmung der HIV-Replikation gemessen wird.

Zunächst erfolgt eine Behandlung von HIV-infizierten humanen T-Lymphomzellen bzw. HIV-infizierten humanen fetalen Lungenzellen mit der zu untersuchenden Verbindung in Verdünnungsreihe. Anschließend erfolgt eine Quantifizierung der Virusmenge im Überstand durch Neuinfektion von humanen fetalen Lungenzellen. Die Abnahme der entstandenen HIV-positiven Zellkolonien gilt als direktes Maß für die antivirale Wirksamkeit der untersuchten Verbindung.

In der folgenden Tabelle sind diejenigen Konzentrationen angegeben, die eine 50 %-ige Hemmung der Virusreplikation bewirken ($IC_{50}$-Werte).

Tab.: Zusammenstellung der $IC_{50}$-Werte bezüglich der chronisch infizierten humanen fetalen Lungenzellen.

| Substanz (Bsp.Nr.) | $IC_{50}$-Wert (/ug/ml) |
|---|---|
| 1 | 30 |
| 2 | 1 |
| 3 | 12 |

## Patentansprüche

**1.** Verbindungen der Formel I

$$R^4-A-Y-\underset{\displaystyle O^-}{\underset{|}{\overset{\displaystyle O}{\overset{\|}{P}}}}-O-R^2-\overset{+}{N}(R^3)_3 \qquad (I),$$

in der

R4 einen geradkettigen oder verzweigten, gesättigten oder ungesättigten aliphatischen Rest mit 7-28 Kohlenstoffatomen bedeutet,

A die Gruppe

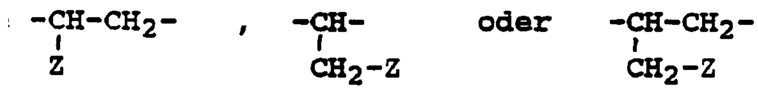

bedeutet, und

Z eine $C_1$-$C_{20}$-Alkoxycarbonyl-, $C_1$-$C_{20}$-Alkylcarbonyl-, $C_1$-$C_{20}$-Alkylaminocarbonyl-, $C_1$-$C_{20}$-Alkylmercapto-, $C_1$-$C_{20}$-Alkansulfinyl-, $C_1$-$C_{20}$-Alkansulfonyl- oder $C_8$-$C_{15}$-Alkoxygruppe darstellt, wobei für den Fall, daß $R^4$ eine geradkettige Alkylgruppe mit 7-18 C-Atomen bedeutet, Z auch eine unverzweite Alkylgruppe mit 9-13 C-atomen sein kann,

$R^2$ eine geradkettige oder verzweigte Alkylenkette mit 2-4 Kohlenstoffatomen,

$R^3$ Wasserstoff oder eine $C_1$-$C_6$-Alkylgruppe

Y ein Sauerstoff- oder Schwefelatom

bedeuten, wobei die Verbindungen bezüglich der Definition $R^4$-A- nicht merh als maximal 33 Kohlenstoffatome besitzen, sowie deren pharmakologisch unbedenkliche Salze und optische Isomere, mit Ausnahme der Verbindungen 2-Decyl-tetradecyl-1-phosphorsäure-monocholinester und 2-Tetradecyl-octadecano-1-phosphocholin.

2. Verbindungen der Formel I gemäß Anspruch 1, in der $R^4$ einen Alkylrest mit 9-17 Kohlenstoffatomen bedeutet.

3. Verbindungen der Formel I gemäß Anspruch 1, in der $R^4$ eine $C_7$-$C_{14}$-Alkylgruppe ist und Z eine $C_8$-$C_{15}$-Alkylmercapto-, $C_8$-$C_{15}$-Alkoxy-, $C_8$-$C_{15}$-Alkoxycarbonyl-, $C_8$-$C_{15}$-Alkylcarbonyl, $C_8$-$C_{15}$-Alkylaminocarbonyl- oder $C_8$-$C_{15}$-Alkylcarbonylaminogruppe bedeutet.

4. Arzneimittel, enthaltend mindestens eine Verbindung gemäß Anspruch 1, 2 oder 3 neben üblichen Träger- und Hilfsstoffen.

5. Verwendung von Verbindungen gemäß Anspruch 1, 2 oder 3 zur Herstellung von Arzneimitteln zur Behandlung von viralen oder retroviralen Infektionen.

## Claims

1. Compounds of the formula I

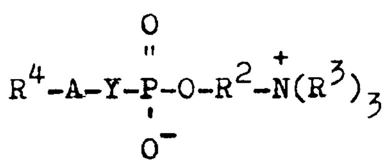

(I)

in which $R^4$ signifies a straight-chained or branched, saturated or unsaturated aliphatic radical with 7 - 28 carbon atoms, A signifies the group

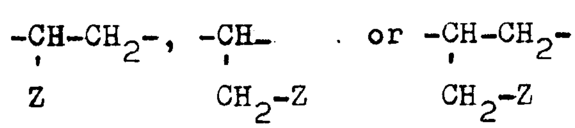

and Z represents a $C_1$-$C_{20}$-alkoxycarbonyl, $C_1$-$C_{20}$-alkylcarbonyl, $C_1$-$C_{20}$-alkylaminocarbonyl, $C_1$-$C_{20}$-alkylmercapto, $C_1$-$C_{20}$-alkanesulphinyl, $C_1$-$C_{20}$-alkanesulphonyl or $C_8$-$C_{15}$-alkoxy group, whereby, for the case that $R^4$ signifies a straight-chained alkyl group with 7 - 18 C-atoms, Z can also be an unbranched alkyl group with 9 - 13 C-atoms, $R^2$ signifies a straight-chained or branched alkylene chain with 2 - 4 carbon atoms, $R^3$ hydrogen or a $C_1$-$C_6$-alkyl group, Y an oxygen or sulphur atom, whereby, with regard to the definition of $R^4$-A-, the compounds possess not more than maximum 33 carbon atoms, as well as their

pharmacologically compatible salts and optical isomers, with the exception of the compounds 2-decyltetradecyl-1-phosphoric acid monocholine ester and 2-tetradecyl-octadecano-1-phosphocholine.

2. Compounds of the formula I according to claim 1, in which $R^4$ signifies an alkyl radical with 9 - 17 carbon atoms.

3. Compounds of the formula I according to claim 1, in which $R^4$ is a $C_7$-$C_{14}$-alkyl group and Z signifies a $C_8$-$C_{15}$-alkylmercapto, $C_8$-$C_{15}$-alkoxy, $C_8$-$C_{15}$-alkoxycarbonyl, $C_8$-$C_{15}$-alkylcarbonyl, $C_8$-$C_{15}$-alkylaminocarbonyl or $C_8$-$C_{15}$-alkylcarbonylamino group.

4. Medicaments containing at least one compound according to claim 1, 2 or 3, besides usual carrier and adjuvant materials.

5. Use of compounds according to claim 1, 2 or 3 for the preparation of medicaments for the treatment of viral or retroviral infections.

## Revendications

1. Composés de formule générale I

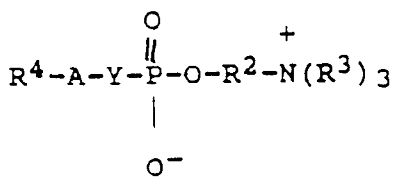

(I)

dans laquelle

$R^4$ représente un groupe aliphatique saturé ou insaturé, à chaîne droite ou ramifiée, comportant 7-28 atomes de carbone,

A représente le groupe

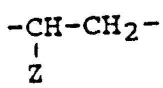 , 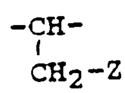 ou 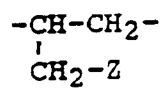

et

Z représente un groupe alcoxy($C_1$-$C_{20}$)-carbonyle, alkyl($C_1$-$C_{20}$)-carbonyle, alkyl($C_1$-$C_{20}$)-aminocarbonyle, alkyl($C_1$-$C_{20}$)-mercapto, alcane($C_1$-$C_{20}$)-sulfinyle, alcane($C_1$-$C_{20}$)-sulfonyle ou alcoxy($C_8$-$C_{15}$), Z pouvant également représenter, dans le cas où $R^4$ représente un groupe alkyle à chaîne droite comportant 7-18 atomes de carbone, un groupe alkyle non ramifié, comportant 9-13 atomes de carbone,

$R^2$ représente un groupe alkylène à chaîne droite ou ramifiée, comportant 2-4 atomes de carbone,

$R^3$ représente un atome d'hydrogène ou un groupe alkyle($C_1$-$C_6$),

Y représente un atome d'oxygène ou de soufre,

les composés selon la définition de $R^4$-A- ne comportant pas plus de 33 atomes de carbone au maximum, ainsi que leurs sels pharmacologiquement acceptables et leurs isomères optiques, à l'exception des composés qui sont l'ester de monocholine de l'acide 2-décyl-tétradécyl-1-phosphorique et la 2-tétradécyl-octadécano-1-phosphocholine.

2. Composés de formule I selon la revendication 1, dans lesquels $R^4$ représente un groupe alkyle comportant 9-17 atomes de carbone.

3. Composés de formule I selon la revendication 1, dans lesquels $R^4$ représente un groupe alkyle($C_7$-$C_{14}$) et Z un groupe alkyl($C_8$-$C_{15}$)-mercapto, alcoxy($C_8$-$C_{15}$), alcoxy($C_8$-$C_{15}$)-carbonyle, alkyl($C_8$-$C_{15}$)-carbonyle, alkyl($C_8$-$C_{15}$)-aminocarbonyle ou alkyl($C_8$-$C_{15}$)-carbonylamino.

4. Médicament, contenant au moins un composé selon la revendication 1, 2 ou 3, en plus de véhicules et adjuvants usuels.

5. Utilisation de composés selon la revendication 1, 2 ou 3, pour la préparation de médicaments destinés au traitement d'infections virales ou rétrovirales.